Europäisches Patentamt

European Patent Office

Office européen des brevets

(11) Publication number: **0 266 940 B1**

# EUROPEAN PATENT SPECIFICATION

(45) Date of publication of patent specification: **16.12.92**    (51) Int. Cl.⁵: **A61K 37/66**

(21) Application number: **87309312.4**

(22) Date of filing: **21.10.87**

The file contains technical information submitted after the application was filed and not included in this specification

(54) **Use of recombinant human alpha interferon for the manufacture of a medicament for the treatment of AIDS virus.**

(30) Priority: **22.10.86 US 921922**

(43) Date of publication of application:
**11.05.88 Bulletin 88/19**

(45) Publication of the grant of the patent:
**16.12.92 Bulletin 92/51**

(84) Designated Contracting States:
**AT BE CH DE ES FR GB GR IT LI LU NL SE**

(56) References cited:

**Interferon, vol.4, 1985 chapter 19, pages 397-316 E.C. Borden et al.:"Studies with interferons produced by recombinant DNA technology in patients with cancer"**

**Interferon, vol.4, 1985, chapter 21, pages 325-335 B.G. Leventhal: "Treatment of virus-associated tumors and papillomas with interferons"**

(73) Proprietor: **SCHERING CORPORATION
2000 Galloping Hill Road
Kenilworth New Jersey 07033(US)**

(72) Inventor: **Feinberg, Judith
21 Boyden Parkway
Maplewood New Jersey 07040(US)**

(74) Representative: **Ritter, Stephen David et al
Mathys & Squire 10 Fleet Street
London EC4Y 1AY(GB)**

EP 0 266 940 B1

EP 0 266 940 B1

Chemical Abstracts, vol. 105, no. 5, 4th August 1986, page 572, abstract no. 40857c, Columbus Ohio, US; J.K. Yamamoto et al.:"Human alpha-and beta-inferon but not gamma-suppress the in vitro replication of LAV, HTLV-III, and ARV-2" & J. Interferon Res. 1986, 6(2), 143-52

Chemical Abstracts, vol. 104, no. 11, 17th March 1986, page 517, abstract no. 86810q, Columbus, Ohio, US; A. Dolei et al.: "Direct and cellmediated inhibition of the human T lymphotropic virus III (HTLV-III) by interferons", & SERONO SYMP. PUBL. RAVEN PRESS 1985, 24(Interferon syst), 183-7

The New England J. of Med., vol. 308, no. 18, 1983, pages 1071-1076 S.E. Krown et al.: "Preliminary observations on the effect of recombinant leukocyte A interferon in homosexual men with Kaposi's sarcoma"

Proc.Am.Soc.Clin.Oncol., vol.3, 1984, page 63, abstract C-245 A.Rios et al.: "The use of lymphoblastoid interferon HuIFN alpha(Ly) in the treatment of acquired immunodeficiency syndrome (AIDS) related Kaposi's sarcoma (KS)

Proc.Am.Soc.Clin.Oncol., vol.2, 1983, page 53, abstract C-206 P. Volberding et al.: "Therapy of Kaposi's sarcoma (KS) in acquired immune deficiency (AIDS) with alpha-2 recombinant interferon"

Proc.Am.Soc.Clin.Oncol., vol. 3, 1984, page 55, abstract C-211 F.X. Real et al.: "Treatment of Kaposi's sarcoma (KS) with recombinant leukocyte a interferon" (rIF-alphaA)

Lancet, 1985, Ho et al., pp. 602-604

Annals of Int. Med., 100, 1984, pp. 671-676, Groopman et al.

## Description

This invention relates to the manufacture of a medicament for treating human immunodeficiency virus (HIV), the retrovirus which is known to cause acquired immunodeficiency syndrome (AIDS). The virus is also known as human lymphotropic virus III (HTLV-III), lymphadenopathy - associated virus (LAV) or AIDS - associated retrovirus (ARV).

AIDS is presumed to be caused by HTLV-III, a retrovirus that specifically infects T-helper lymphocytes, eventually resulting in lymphopenia and deranged cell-mediated immunity. HTLV-III has been isolated from patients with AIDS and the AIDS prodome as reported by Gallo et al., Science, 224, 500 (1984) and Popovic et al., Science, 224, 497 (1984). Also, Redfield et al., JAMA, 253, 11 (1985) isolated HTLV-III from healthy individuals belonging to defined risk groups. According to the Center for Disease Control (CDC), Atlanta, Georgia those at increased risk of HTLV-III infection in the adult population include homosexual or bisexual men, intravenous drug users, haemophiliacs who have received factor concentrates, heterosexuals having contact with individuals in these groups and recipients of transfusions from donors in these groups, MMWR, 34 No. 18, May 10, 1985. A number of seroepidemiologic studies in healthy individuals belonging to high-risk groups indicate widespread exposure to the virus, both in industrialized nations and in developing nations, e.g., MMWR, 33 (1984); Multicenter AIDS Cohort Study, 25th ICAAC, Abstract No. 740 (1985); Weiss et al., 25th ICAAC, Abstract No. 734 (1985); Melbye et al., Br. Med. J. 289 (1984); Alos et al., 25th ICAAC, Abstract No. 737 (1985) and Kreiss et al., 25th ICAAC, Abstract No. 227 (1955). Extrapolations made from the data in these studies indicate that as many as 500,000 to 1,700,000 Americans are seropositive, [Landesman et al., NEJM, 312, 521 (1985); Hardy et al., JAMA, 253:2 (1985); Sivak et al., NEJM 313:21 (1985)]. In addition, both antibody positive and antibody negative individuals in high-risk groups have been shown to be culture positive for HTLV-III, Jaffe et al., Ann. Int. Med. 102:5 (1985) and Salahuddin et al., Lancet, 1418, (Dec. 22/29, 1984).

Clearly, AIDS is a significant and fast growing health problem. To date no significant means to treat the AIDS causing virus has emerged.

Ho et al., Lancet (1985), in in vitro experiments has shown that interferon alfa-2a is active against HTLV-III. No in vivo tests were performed.

Interferons are a family of proteins which exhibit antiviral activity against certain viruses and anticancer activity against certain cancers. There are three types of interferons; alpha or leukocyte interferon, beta or fibroblast interferon and gamma or immune interferon. Human alpha interferon is a naturally occurring mixture of at least eleven components including those designated alpha-1 interferon and alpha-2 interferon. Alpha interferon exhibiting biological properties similar to those of naturally occurring human leukocyte interferon can be made by recombinant methods.

A number of alpha interferon species or components are known and are usually designated by a numeral after the Greek letter alpha, and all are contemplated for use in this invention. Thus, the species designated human alpha-1 interferon is contemplated for use in this invention and human alpha-2 interferon which includes human-alpha-2a and human alpha-2b interferon, and under USAN, designated Interferon Alfa-2 which includes Interferon Alfa-2a and Interferon Alfa-2b, is also contemplated for use in this invention. "Human interferon alfa-2" is used herein when referring to human alpha-2 interferon. Interferon alfa-2b is the preferred species of human interferon alfa-2.

Interferon alfa-2 can be produced in bacteria using recombinant techniques as disclosed in Rubenstein, Biochem. Biophys, Acta, 695, 5-16 (1982). In addition, interferon alfa-2 may be prepared by recombinant-DNA methods disclosed by Nagata et al., Nature, 284, 316-320 (1980), European Patent 32,134 and U.S. Patent No. 4,289,690. Various interferon alfa-2 species are disclosed in U.S. Patent 4,503,035. The preferred human interferon alfa-2b used in this invention is also denoted (hIFN-α2b).

This invention relates to the use of recombinant interferon alpha-2, preferably human recombinant DNA interferon alfa-2 (hIFN-α2) in the manufacture of a medicament for use as an anti-HTLV-III agent in the treatment of patients having HTLV-III viremia or in the maintenance in an HTLV-III aviremic state of a previously HTLV-III viremic patient.

Medicaments so manufactured may be used for treating patients infected with the retrovirus (HTLV-III) which causes AIDS using certain alpha interferons at high doses, i.e., from 5 to 75 million International units of alpha interferon administered by injection e.g., subcutaneously in one administration, for the number of days and at a frequency needed to maintain assays of the virus at sufficiently low levels to be considered inactive.

As used herein, the term "HTLV-III" means the RNA retrovirus which causes AIDS. The term "alpha interferon" means recombinant alpha-1 interferon and recombinant alpha-2 interferon, sometimes referred to as interferon alfa-2. In most instances this invention will be described in the following discussion using "human recombinant interferon alfa-2b" or "hIFN-α2b".

"Reverse transcriptase" is a viral enzyme that is a virus coded, RNA dependent DNA polymerase.

The presence of the enzymatic activity of this reverse transcriptase is used as a marker for the presence of the AIDS virus.

The AIDS retrovirus can be identified in culture by its cytopathic effect (CPE) as it has the property of causing the formation of giant syncytia which appears to be due to the fusion of cells. These syncytia, which appear as fragile spheres containing multiple nuclei, are characteristic of the presence of the AIDS retrovirus.

According to this invention, people with AIDS retrovirus infections, i.e. those with HTLV-III in their blood, are administered from 5 to 75 million International Units of recombinant interferon alfa-2b parenterally, once a day, until the cytopathic effect (CPE) and reverse transcriptase (RT) values both indicate two successive negative cultures. Conversely, other indications of the virus activity, such as p24 antigen in the serum, antigen capture by conventional, commercially available techniques, or gene amplification by standard bioligic techniques may be used as end points for the purpose of this invention. Then the patient is administered a sufficient amount of interferon either every day or every other day or any suitable period as determined by the attending clinician to maintain the culture negative state. This maintenance is a key element in the treatment regimen because unless the maintenance regimen is followed, the HTLV-III may again become present in sufficient numbers to show a positive culture when tested for CPE and RT.

The RT values are determined as follows:

Virus particles are precipitated from cell-free supernatant as follows: 0.3 ml of 4M NaCl and 3.6 ml of 30 percent (weight to volume) polyethylene glycol (Carbowax 6000) are added to 8 ml of harvested culture fluids and the suspension is placed on ice overnight. The suspension is centrifuged in a Sorvall RC-3 centrifuge at 2000 rev/min at 4°C for 30 minutes. The precipitate is resuspended in 300 $\mu$l of 50 percent (by volume) glycerol (25 mM tris-HCl, pH 7.5, 5 mM dithiothreitol, 150 mM KCl, and 0.025 percent Triton® X-100). Virus particles are disrupted by addition of 100 $\mu$l of 0.9 percent Triton® X-100 to 1.5M KCl. Reverse transcriptase assays are performed as previously described [Poiesz et al., Proc. Natl. Acad. Sci. USA 77, 7415 (1980); Yashida, et al., Proc. Natl. Acad. Sci. USA, 79, 2031 (1982); Gallo et al., Hematopoietic Mechanisms, Clarkson et al., eds., 5, 671 (1978) Cold Spring Harbor Press, Cold Spring Harbor, NY.] and expressed in counts per minute per milliliter of culture medium.

Triton® X-100 is octoxynol-9 and is available from Rohm and Haas. Carbowax®-6000 is polyethylene glycol with a molecular weight between 7000 and 9000 and is available from Union Carbide.

A discussion of cytopathic effects is found, for example, in Popovic et al., Science, 224, 497 (1984) and Gallo, et al., Science 224, 500 (1984).

The preferred mode of administering the interferon alfa-2 in the high doses needed is by subcutaneous injection. This mode of administration is advantageous because the patient can self-treat.

For subcutaneous administration, liquid injectable pharmaceutically acceptable compositions are used. Such compositions can be prepared, for example, by diluting lyophilized hIFN-$\alpha$2b with sterile pyrogen free water for injection to produce a sterile solution containing the appropriate concentration of hIFN-$\alpha$2b, buffers and other additives known in the art.

The amount of alpha interferon administered can vary between 5 to 75 million International units per dose. The preferred amount is about $35 \times 10^6$ IU.

High doses as contemplated for use in this invention generally result in side effects which are associated with interferon administration, including blood cell count reduction. However, upon dose reduction and/or cessation of treatment, the side effects usually disappear within 1 to 3 days. Treatment can then be resumed. Dose reduction, interruption, and resumption of the treatment is as determined in the judgement of the attending clinician.

Generally, the dosage regimen comprises administration of human recombinant interferon alfa-2 daily for about twelve weeks with $35 \times 10^6$ IU, then every other day with e.g., 25 or $35 \times 10^6$ IU, or even less, depending on the dosage required to maintain a negative culture as evidenced by tests measuring the CPE and RT values in the patient's blood.

The following describes a clinical test demonstrating is invention using human recombinant interferon alfa-2b.

METHODS AND MATERIALS

Patient Population

Enter about 50 to 80 patients into a randomized placebo-controlled trial designed to treat HTLV-III viremia with human recombinant interferon alfa-2b. Select patients from asymptomatic males at risk for HTLV-III infection between 18 and 60 years old who test positive for the presence of HTLV-III in the blood by antibody determination. The high risk group is homosexual or bisexual men or haemophiliacs, but not intravenous drug abusers.

HTLV-III seropositivity is measured on two separate occasions by ELISA technique, i.e. a commonly used test for screening sera for the presence of antibodies to HTLV-III. Kits for this test are manufactured by Abbott Laboratories, Travenol Laboratories, Biotech and Litton. The results are confirmed by the Western blot technique carried out as follows:

A 300-$\mu$l sample of virus containing 10 to 100 $\mu$g of protein is placed in 300 $\mu$l of sodium dodecyl sulfate sample buffer (25 mM Tris, 10% glycerol, 2.3% sodium dodecyl sulfate) containing 7.0 $\times$ $10^{-6}$ M 2-mercapto ethanol (5%). The solution is boiled for 2 min. and then placed onto a 3 to 27% gradient polyacrylamide gel (15 by 0.15 cm). The sample is then electrophoresed for approximately 16 h at 50 V. The electrophoresed proteins are then transferred to a nitrocellulose filter, and the filter is incubated with TN buffer (10 mM Tris, 155 mM NaCl, pH 7.4) containing 5% milk protein for 30 min. at room temperature to saturate all protein-binding sites on the nitrocellulose. The nitrocellulose is next washed with TN-TN buffer [TN buffer, 0.3% Tween® 20 (a mixture of laurate esters of sorbitol and sorbitol anhydrides condensed with approximately 20 moles ethylene oxide, available from ICI Americas, Wilmington, Delaware), 0.05% Nonidet P-40 {$\alpha$-[1,1,3,3-tetramethylbutyl)phenyl]-$\omega$ -hydroxy-poly(oxy-1,2-ethanediyl) available from Sigma Chemical Company, St. Louis, Missouri} for 5 min. to remove excess milk proteins. The nitrocellulose is then cut into approximately 1 cm strips, and each strip is identified using indelible ink. Care should be taken to keep the nitrocellulose moist throughout the entire procedure. The strips are then placed into a slot incubation tray or screw-top test tube with a 1:1,000 dilution of the test serum and allowed to incubate at room temperature for 2 to 16 hours. The strips are then washed twice for 5 min. with TN-TN buffer and then once for 5 min. with TN-T buffer (TN, 0.3% Tween® 20). The strips may be combined at this point. They are next incubated for 1 to 3 hours at room temperature with 200,000 dpm of $^{125}$I-protein A per ml in TN-T buffer containing 3% bovine serum albumin. The strips are then washed three times for 5 min. each with TN-TN buffer containing 10 mM EDTA and then once for 5 min. with TN buffer. The strips are then allowed to air dry, and autoradiography is performed at -70° C with an intensifying screen.

Patients are ineligible if they have had systemic corticosteroid, antineoplastic or antiviral therapy within 6 months, prior interferon therapy or a concurrent infection, a history of opportunistic infections or diffuse lymphadenopathy.

Study Design

Prior to enrollment into the study patients are apprised of the procedures involved and those willing to comply with the study design have the following procedures performed.

1. Confirmatory Western blot testing.
2. Culture of peripheral blood mononuclear cells for HTLV-III in PHA-simulated blasts and assay of RT activity.
3. Complete history including assessment of risk factors.
4. Complete physical, with particular attention to integument, tongue, lymph nodes, and neurologic function.

Asymptomatic individuals with documented viremia then undergo the following laboratory examinations:

1. Complete blood count, total bilirubin, AST, ALT, alkaline phosphatase, blood urea nitrogen (BUN), creatinine, fasting glucose, and urinalysis.
2. Total lymphocyte count and helper and suppressor T-cell subsets.
3. Ability to mount a proliferative response to tetanus toxoid in vitro.

The study is a randomized, double-blind, placebo controlled design and all qualified patients are treated for a minimum of 12 weeks. The patients are stratified for the ability to mount an adequate proliferative lymphocyte response to tetanus toxoid in vitro (this is an indication of lowered immunity), and are initially divided into two groups;

(a) those receiving daily therapy with 35 million International Units of alpha interferon for 12 or more weeks, and (b) those receiving placebo therapy for 12 or more weeks. The placebo consists of the vehicle used in the formulation of the alpha interferon injectable. It contains dibasic sodium phosphate, monobasic sodium phosphate monohydrate, glycine, human albumin, and water.

Patients who are aviremic after up to twenty four weeks of therapy are further randomized to continue treatment at the same dose on a daily or every other day treatment schedule for an additional three months. The placebo treated subjects who become aviremic during the treatment phase are also followed for an additional three months as untreated controls.

Because of the high doses used, some patients exhibit toxic effects. When this occurs, the daily dose is decreased from 35 million International Units to 25 million International Units to 15 million International Units to 10 million International Units or less, as needed in the clinician's judgement. When an acceptable level of toxicity is achieved, the dosage is adjusted upward until the proper balance, in the clinician's judgement, is achieved between toxicity and reduced HTLV-III in the blood.

Maintenance

Patients who are not aviremic by the end of the 24 week treatment are withdrawn from the study.

Patients having two consecutive negative cultures remain in the study to determine which dosage regimen will maintain the negative cultures.

A "negative culture" is defined as negative cytopathologic affect (CPE) and a negative RT assay, all other cultures are positive. A negative CPE is absence of cytopathologic effect consisting of a syncytia formation score of zero rated on a scale of 0 to 3 + .

A negative RT assay is a peak reverse transcriptase titer of less than 1000. As noted previously, other techniques may be used to detect a "culture-negative" state and still fall under the guidelines of this invention.

It is expected as a result of the study that negative cultures will occur in the majority of patients receiving daily doses of 35 million International units of human recombinant interferon alfa-2 and that doses of from 15 to 35 million International Units of human recombinant interferon alfa-2 administered daily every other day, with each administration a single dose, will be sufficient to maintain negative cultures.

"Carbowax" and "Triton" are Registered Trade Marks.

## Claims

1. The use of recombinant human alpha interferon in the manufacture of a medicament for use as an anti-HTLV-III agent in the treatment of patients having HTLV-III viremia, or in the maintenance in an HTLV-III aviremic state of a previously HTLV-III viremic patient.

2. The use of claim 1 wherein the alpha interferon is recombinant human alpha-2 interferon.

3. The use of claim 2 wherein the alpha-2 interferon is recombinant human interferon alfa-2.

4. The use of claim 3 wherein the recombinant human interferon is recombinant human interferon alfa-2b.

5. The use of claim 4 wherein the said medicament is suitable for administration at a dose rate from 5 million International units to 75 million International units of human interferon alfa-2b per day.

6. The use of claim 5 wherein the said medicament is suitable for administration at a dose rate of about 35 million International units of human interferon alfa-2b per day.

7. The use claimed in claim 1 wherein said medicament is suitable for subcutaneous administration.

8. The use claimed in any preceding claim wherein the recombinant human interferon alpha 2 is manufactured in the form of a pharmacuetical composition for treating patients infected with HTLV-III comprising a sufficient amount of recombinant human interferon alpha-2 to be effective against HTLV-III in a suitable pharmaceutically acceptable carrier.

## Patentansprüche

1. Verwendung von rekombiniertem menschlichen alpha-Interferon bei der Herstellung eines Arzneimittels zur Verwendung als Anti-HTLV-III-Mittel bei der Behandlung von Patienten mit HTLV-III-Virämie oder bei der Aufrechterhaltung eines HTLV-III-avirämischen Zustandes eines zuvor HTLV-III-virämischen Patienten.

2. Verwendung nach Anspruch 1, worin das alpha-Interferon rekombiniertes Human-alpha-2-Interferon ist.

3. Verwendung nach Anspruch 2, worin das alpha-2-Interferon rekombiniertes Human-Interferon-alfa-2 ist.

4. Verwendung nach Anspruch 3, worin das rekombinierte Human-Interferon rekombiniertes Human-Interferon-alfa-2b ist.

5. Verwendung nach Anspruch 4, worin das Arzneimittel für eine Verabreichung in einer Dosisrate von 5 Millionen Internationalen Einheiten bis zu 75 Millionen Internationalen Einheiten Human-Interferon-alfa-2b pro Tag geeignet ist.

6. Verwendung nach Anspruch 5, worin das Arzneimittel für eine Verabreichung in einer Dosisrate von etwa 35 Millionen Internationalen Einheiten Human-Interferonalfa-2b pro Tag geeignet ist.

7. Verwendung nach Anspruch 1, worin das Arzneimittel für eine subkutane Verabreichung geeignet ist.

8. Verwendung nach irgendeinem der vorhergehenden Ansprüche, worin das rekombinierte Human-Interferon-alpha-2 in Form einer pharmazeutischen Zusammensetzung zur Behandlung von mit HTLV-III infizierten Patienten angefertigt wird, die in einem pharmazeutisch annehmbaren Träger eine ausreichende Menge an rekombiniertem Human-Interferon-alpha-2 enthält, damit dieses gegen HTLV-III wirksam ist.

**Revendications**

1. Utilisation de l'interféron humain recombinant alpha pour la fabrication d'un médicament destiné à être utilisé comme agent anti-HTLV-III dans le traitement de malades présentant une virémie HTLV-III, ou pour le traitement d'entretien d'un patient précédemment virémiques à HTLV-III dans un état avirémique vis-à-vis du HTLV-III.

2. Utilisation selon la revendication 1, dans laquelle l'interféron alpha est un interféron humain recombinant alpha-2.

3. Utilisation selon la revendication 2, dans laquelle l'interféron alpha-2 est l'interféron humain recombinant alfa-2.

4. Utilisation selon la revendication 3, dans laquelle l'interféron humain recombinant est l'interféron humain recombinant alfa-2b.

5. Utilisation selon la revendication 4, dans laquelle ledit médicament convient à une administration à une posologie allant de 5 millions d'Unités Internationales à 75 millions d'Unités Internationales d'interféron humain alfa-2b par jour.

6. Utilisation selon la revendication 5, dans laquelle ledit médicament convient à une administration à une posologie d'environ 35 millions d'Unités Internationales de l'interféron humain alfa-2b par jour.

7. Utilisation selon revendication 1, dans laquelle ledit médicament convient à une administration sous-cutanée.

8. Utilisation selon l'une l'une quelconque des revendications précédentes, dans laquelle l'interféron humain recombinant alpha-2 est fabriqué sous la forme d'une composition pharmaceutique destinée au traitement de malades infectés par le virus HTLV-IIIn comprenant une quantité de l'interféron humain recombinant alpha-2 suffisante pour être efficace contre le virus HTLV-III dans un excipient approprié, pharmaceutiquement acceptable.